Europäisches Patentamt

European Patent Office

Office européen des brevets

(19)

(11) Veröffentlichungsnummer: **0 198 912**
**B1**

(12) **EUROPÄISCHE PATENTSCHRIFT**

(45) Veröffentlichungstag der Patentschrift: **14.03.90**

(51) Int. Cl.⁵: **A 61 L 25/00**

(21) Anmeldenummer: **85905616.0**

(22) Anmeldetag: **24.10.85**

(86) Internationale Anmeldenummer:
**PCT/EP85/00561**

(87) Internationale Veröffentlichungsnummer:
**WO 86/02560 09.05.86 Gazette 86/10**

(54) VERFAHREN ZUR MECHANISCHEN ZERRUETTUNG VON AUSPOLYMERISIERTEN KUNSTSTOFFEN.

(30) Priorität: **29.10.84 DE 3439533**

(43) Veröffentlichungstag der Anmeldung:
**29.10.86 Patentblatt 86/44**

(45) Bekanntmachung des Hinweises auf die
Patenterteilung:
**14.03.90 Patentblatt 90/11**

(84) Benannte Vertragsstaaten:
**AT BE CH DE FR GB IT LI NL SE**

(56) Entgegenhaltungen:
**EP-A-0 002 064**
**EP-A-0 007 287**
**DE-A-2 947 885**

(73) Patentinhaber: **MERCK PATENT GESELLSCHAFT
MIT BESCHRÄNKTER HAFTUNG
Frankfurter Strasse 250 Postfach 4119
D-6100 Darmstadt (DE)**

(72) Erfinder: **HOFF, Günter
Alpenblick 42
D-7758 Daisendorf (DE)**
Erfinder: **OHNSORGE, Jochen
Kaiser-Friedrich-Allee 4
D-5100 Aachen (DE)**

**Beschreibung**

Die Erfindung betrifft ein Verfahren zur mechanischen Zerrüttung von auspolymerisierten Kunststoffen, insbesondere von Knochenzementen.

Ein zentrales Problem der Implantation von Gelenkendoprothesen besteht in der Verankerung der Prothese im Knochenlager. Es ist bekannt, dafür Knochenzemente auf Acrylatbasis einzusetzen, die durch Mischen und Auspolymerisieren von pulverförmigen Präpolymeren mit flüssigen Monomeren, die Initiatoren und Beschleuniger für die Polymerisation enthalten, hergestellt werden. Diese dienen in der orthopädischen Chirurgie zur sicheren und schnellen Primärfixierung des Gelenkimplantates am Knochen, wodurch dem Patienten eine Frühbelastung des Gelenkes ermöglicht wird. Der in der Anrührphase zunächst noch teigige, langsam aushärtende Knochenzement füllt den Raum zwischen Knochen und Gelenkimplantat nahezu fugenlos aus, erhält jedoch durch den fortlaufenden Polymerisationsvorgang sehr schnell die volle Festigkeit.

Diese Art der Fixierung von Gelenkendoprothesen am Knochen mittels auspolymerisiertem Knochenzement ist heute eine weltweit angewandte und anerkannte Operationstechnik in der orthopädischen Chirurgie. Probleme wie zu hohe Energiefreisetzung und damit zu hohe Temperatur bei der Polymerisation, die zur Nekrotisierung des Gewebes führt, sind ebenso wie die Fragen der ausreichenden Bio-kompatibilität des Knochenzementes prinzipiell gelöst.

Langfristig kann es jedoch zu Auslockerungen des Metall-implantates oder des Kunststoffimplantates im Knochenzementlager kommen, weswegen Prothesenwechsel mit Entfernung des Knochenzementes erforderlich werden. Die Entfernung des Knochenzementes aus Röhrenknochen ist technisch sehr schwierig und bedeutet eine lange Operationszeit bei Reoperationen von Gelenkendoprothesen, wodurch die überwiegend alten Patienten erheblich gefährdet sind durch lange Operationszeiten, lange Blutungszeiten der unverschlossenen Weichteile und erhöhte Infektionsgefahr durch lange Wundöffnungszeiten. Die Bedeutung dieses Problems läßt sich daran ermessen, daß bis zu 20% aller heute durchgeführt Gelenkprothesenoperationen Reoperationen sind.

Es bestand deshalb die Aufgabe ein Verfahren zu finden, mit dem ein auspolymerisierter Kunststoff mechanisch so zerrütet werden kann, daß er im Falle einer notwendig werdenden Reoperation leicht und schnell aus dem Knochen entfernt werden kann.

Diese Aufgabe wurde durch die vorliegende Erfindung gelöst. Es wurde nämlich gefunden, daß in die Polymermatrix eingelagerte Störkörper, deren Schallwellenwiderstand sich von dem der Polymermatrix unterscheidet, mit Hilfe von akustischen Wellen so angeregt werden können, daß an den akustischen Grenzflächen Zug- und Druckspannungen erzeugt werden, die zum Zerfall der Polymermatrix führen.

Gegenstand der Erfindung ist daher ein Verfahren zur mechanischen Zerrüttung von auspolymerisierten Kunststoffen, das dadurch gekennzeichnet ist, daß vor dem Aushärten des Polymeren akustische Störkörper mit einem von dem des Polymeren unterschiedlichen Schallwellenwiderstand eingelagert werden, und daß durch selektive Anregung der Störkörper durch akustische Wellen die Polymermatrix zerstört wird.

Gegenstand der Erfindung ist auch ein Knochenzement auf der Basis von physiologisch verträglichen Polymeren, insbesondere von aus Präpolymeren und Monomeren hergestellten Polyacrylaten und/oder Polymethacrylaten, der dadurch gekennzeichnet ist, daß in das Polymere 0,5 bis 20 Vol.% akustischer Störkörper einer Dicke von 0,01—2 mm aus einem körperverträglichen Metall eingelagert sind.

Im Stand der Technik, z.B. der EP—A—00 02 064, der EP—A—00 07 287 und der DE—A—29 47 887, sind zwar Knochenzemente beschrieben, in die Fremdstoffe eingelagert sind. Diese Einlagerungen dienen jedoch ausschließlich der Verbesserung des Fe tigkeit des Zement oder dessen besserer Verzahnung mit dem angrenzenden Knochengerüst. Eine Anregung, Einlagerungen als akustische Störkörper zur Zerrüttung der Polymermatrix zu nutzen, ist diesen Publikationen nicht zu entnehmen.

Der Vorteil der vorliegenden Erfindung liegt darin, daß der in dem erfingdungsgemäßen Verfahren eingesetzte Knochenzement durch den Chirurgen in gleicher Weise wie die bisher bekannten Knochenzemente eingesetzt werden kann. In bezug auf Kurz- und Langzeitstabilität wird durch die Einlagerung der Störkörper keine signifikante Verschlechterung bewirkt. Vorteilhaft in bezug auf die Stabilität des Knochenzementes wirkt es sich aus, wenn die Störkörper in den quasistatischen mechanischen Eigenschaften, das heißt in den bei der Normalbeanspruchung (Gehen, Laufen) auftretenden Belastungen maßgebenden Parameter wie z.B. Elastizität und Kompressibilität dem Polymeren möglichst ähnlich sind. Insbesondere dann jedoch, wenn diese Eigenschaften unterschiedlich sind, ist es vorteilhaft, wenn die Störkörper keine scharfen Kanten oder Spitzen besitzen, die zu unerwünschten örtlichen Spannungskonzentrationen und damit zur Rißbildung in der Polymermatrix führen können. Bevorzugt sind daher Störkörper mit abgerundeten Ecken und Kanten, wie zum Beispiel Rotationsellipsoide, Zylinder-scheiben mit abgerundeten Kanten oder Kugeln.

Anstelle von einzelnen Partikeln können jedoch auch Störkörper in der Form von Fäden, Geflechten, Geweben, Folien oder Platten verwendet werden. Besonders vorteilhaft kann z.B. ein netzartiges Gewebe eingesetzt werden, da auf der einen Seite dadurch eine beträchtliche Stabilisierung des Knochenzements erreicht werden kann und auf der anderen Seite im Falle einer Reoperation der Zement sehr effektiv entlang der Knochen-Zement-Kontaktschicht herausgelöst werden kann, wenn das Netz in dieser Grenzregion angeordnet wird.

Größe und Form der Störkörper sollte so bemessen sein, daß einerseits eine optimale Energieaufnahme von den extrakorporal erzeugten akustischen Wellen möglich ist und daß andererseits die Verarbeitung des Knochenzementes nicht wesentlich beeinträchtigt wird. Störkörper einer Größe bzw. Dicke von etwa 0,01 bis etwa 2 mm, insbesondere einer Dicke von etwa 0,1 bis etwa 1 mm, sind daher bevorzugt.

Um eine wirksame Zerrüttung zu erreichen, ist selbstverständlich eine Vielzahl von Störkörpern, möglichst homogen verteilt in der Polymermatrix, notwendig. Dabei ist die Zerrüttung um so wirkungsvoller, je mehr Störkörper enthalten sind. Andererseits werden durch einen zu hohen Gehalt an Störkörpern die mechanischen Eigenschaften des Knochenzements beeinträchtigt. Ein Gehalt von etwa 0,5 bis etwa 20 Vol. %, insbesondere von etwa 3 bis etwa 10 Vol. %, ist daher bevorzugt.

Als Material für die Störkörper wird ein biokompatibles Material eingesetzt, dessen Schallwellenwiderstand, das heißt das Produkt aus Dichte und Schallgeschwindigkeit, sich von dem der Polymermatrix deutlich unterscheidet. Insbesondere werden Materialien eingesetzt, deren Schallwellenwiderstand sich um mindestens den Faktor 1,5 bevorzugt mindestens um den Faktor 1—10, von dem des Polymeren unterscheidet. Der Schallwellenwiderstand der Störkörper kann dabei sowohl größer als auch kleiner als der des Polymeren sein. Als Material sind daher sowohl körperverträgliche Metalle oder Legierungen, wie zum Beispiel $TiAl_5Fe_{2,5}$, CoCrMo oder Tantal, als auch oxidische Körper, insbesondere in der Form von gebrannten Oxidkeramiken, wie zum Beispiel $Al_2O_3$, als auch Glas oder Kohlenstoff einsetzbar. Anstelle dieser dichten Störkörper können jedoch auch Störkörper mit sehr geringer Dichte, wie zum Beispiel Luft- oder Gasblasen, verwendet werden, die in homogener Verteilung beim Anrühren des Zements mit eingerührt werden können oder die aus geeigneten Substanzen, zum Beispiel durch thermische Zersetzung beim Aushärten des Zements, freigesetzt werden können.

Um im Falle der Verwendung von Einzelteilchen eine möglichst umfassende Zerrüttung des Knochenzements über dessen gesamtes Volumen zu erreichen, sollten diese Störkörper möglichst homogen im Knochenzement verteilt sein. Bei sehr schweren, insbesondere metallischen Störkörpern kann es daher zur Verbesserung des Dispergierbarkeit und zur Verminderung von Sedimentation vorteilhaft sein, diese in eine Hülle des Polymeren einzubetten.

Der erfindungsgemäße Knochenzement wird in analoger Weise wie die bekannten Knochenzemente hergestellt. Diese werden so zubereitet, daß etwa zwei Teile eines feinteiligen, einen Polymerisationskatalysator (z.B. Dibenzoyl-peroxid) enthaltenden Präpolymerisats, insbesondere Polymethylmethacrylat oder eines Mischpolymerisats aus Methylacrylat und Methylmethacrylat, mit etwa einem Teil des flüssigen Monomeren, zum Beispiel Acrylsäure- oder Methacrylsäuremethylester oder deren Gemische, das einen Beschleuniger (z.B. Dimethyl-p-toluidin) enthält, zu einer formbaren Masse gemischt werden, die in den Körper implantiert wird und dort aushärtet. Solche Knochenzemente sind zum Beispiel unter dem Warenzeichen Palacos[R] im Handel. Zusätzlich können darin auch pharmakologisch wirksame Substanzen, wie zum Beispiel Antibiotika, eingearbeitet werden oder auch Materialien, die die Verankerung des Knochenzements im Körper erleichtern, wie zum Beispiel resorbierbares Tricalciumphosphat. Zur Herstellung des erfindungsgemäßen Knochenzements wird in einen solchen bekannten Knochenzement zusätzlich die erforderliche Menge an Störkörpern mit eingearbeitet.

In der Verarbeitung und auch in den nach dem Aushärten erzielten mechanischen Eigenschaften ist der erfindungsgemäße Knochenzemente den bekannten Knochenzementen äquivalent. Der Vorteil zeigt sich jedoch dann, wenn eine Reoperation notwendig werden sollte. In diesem Fall können erfindungsgemäß mit extrakorporalen akustischen Wellen an den akustischen Grenzflächen zwischen Störkörper und Polymermatrix Zug- und Druckspannungen erzeugt werden, die zur Auflösung des Verbunds und zum Zerfall der Polymermatrix führen.

Als akustische Wellen können dabei sowohl extrakorporal erzeugte und auf den Knochenzementpropfen fokussierte Stoßwellen eingesetzt werden als auch Ultraschallwellen mit einer auf die Eigenfrequenz der Störkörper abgestimmten Frequenz.

Stoßwellen werden in der medizinischen Therapie bereits eingesetzt zur Zertrümmerung von Harnsteinen. Die dabei benutzten Geräte zur Erzeugung und Fokussierung der Stoßwellen können grundsätzlich auch zur Zerrüttung der erfindungsgemäßen Knochenzemente eingesetzt werden. Damit werden Stoßwellen in einem Druckbereich von etwa 2 kbar erzeugt. Das Profil dieser Stoßweller, die Halbwertsbreiten von etwa $10^{-8}$ Sekunden aufweisen, ist der Geometrie der Störkörper in der Weise agepaßt, daß die Halbwertsbreite der Stoßwelle kleiner ist als die Dicke des Störkörper und die Druckanstiegszeit etwa um den Faktor 10 kleiner ist als die Halbwertsbreite. Vorzugsweise werden Stoßwellen mit Rechteckprofilen verwendet, die Druckanstiegszeiten von weniger als $10^{-7}$ Sekunden aufweisen.

Im Unterschied zu den bei der Harnstein-Zertrümmerung eingesetzten Geräten sind bei der Zerrüttung des erfindungsgemäßen Knochenzements keine aufwendigen und teuren Stereoröntgenortungs- und Patientenpositionierungssysteme erforderlich, sondern es wird für die erfindungsgemäße Aufgabe ein optisch-mechanisches Positionierungssystem vorgesehen. Mit den in orthopädischen Kliniken verfügbaren Röntgen- und Ultraschalldiagnosegeräten wird die Lage des Knochenzementpropfens im Röhren-

knochen bestimmt und auf der Hautoberfläche markiert. Mit einer Dickenmessung des Binde- und Knochengewebes zwischen Hautoberfläche und Knochenzement über den Gesamtumfang der Gelenkendoprothese sind die vom Fokuspunkt der Stoßwellen zu überstreichenden Volumina eindeutig geometrisch zur Hautoberfläche festgelegt. Mit einer Optik, die entweder in das Stoßwellenzeugungssystem integriert oder mit ihm auf einer Wechselschiene fest verbunden wird, kann der Fokuspunkt der Stoßwellen exakt in den zu zerstörenden Knochenzement positioniert werden.

Eine ebenfalls kostengünstige Alternative zu dem optisch mechanischen Positionierungssystem ist ein in die Stoßwellenerzeugung integriertes Ultraschallortungssystem.

Durch eine extrakorporale Stoßwellenbehandlung des Knochenzementes eines Patienten kurz vor der Reoperation seines Implantates, ist die einfache und sichere Entfernung des Knochenzementes gewährleistet.

Anstelle von Stoßwellen können jedoch auch Schallwellen im Ultraschallbereich, das heißt im Frequenzbereich MHz eingesetzt werden. Die Frequenz des Ultraschalls muß auf die Eigenfrequenz der akustischen Störkörper abgestimmt sein. Über Resonanzeffekte der Störkörper erfolgt die Zerrüttung des Knochenzementes. In der Regel werden Frequenzen von etwa 1 bis etwa 100 MHz verwendet, bei einer Amplitude von etwa 0,1 bis etwa 10 bar.

Die Verwendung von Ultraschall hat den Vorteil, daß Ultraschallgeräte sehr viel preiswerter sind als Stoßwellengeräte. Elektroden als teures Verbrauchsmaterial entfallen vollständig und die Druckamplituden sind etwa um den Faktor 1000 geringer. Außerdem könnte das Ultraschallgerät für die Zerstörung des Knochenzements gleichzeitung zur Ortung des Knochenzementpfropfens benutzt werden. Voraussetzung für die Verwendung von Ultraschall ist jedoch, daß alle Störkörper im wesentlichen die gleiche Resonanzfrequenz und somit die gleiche Geometrie besitzen.

In jedem Fall wird jedoch durch eine Anwendung der entsprechenden Methode kurz vor der notwendigen Reoperation die Entfernung des Knochenzements erheblich erleichtert.

Beispiel 1

Polymermatrix:
Polymethylmethacrylat mit Antibiotikazusatz

Störkörper:
Rotationsellipsoid, entstanden durch Rotation einer Ellipse mit den Halbachsen 1 mm und 2 mm um die kleinere Achse, aus dem Material $TiAl_5Fe_{2,5}$

Stoßwelle:
Druckbereich $\geqq$ 1 kbar
Druckanstiegszeit $\leqq 10^{-7}$ sek.
Halbwertsbreite $\leqq 10^{-6}$ sek.

Beispiel 2

Polymermatrix:
Polymethylmethacrylat mit 1 Gew. % Gentamycin

Störkörper:
Chirurgischer Metallfaden in Form eines Netzes mit einer Maschenweite von etwa 5 mm und einer Fadendicke von etwa 200 µm.

Stoßwelle:
Druckbereich ~ 1kbar
Druckanstiegszeit ~ $10^{-8}$
Halbwertsbreite ~ $10^{-7}$

**Patentansprüche**

1. Verfahren zur mechanischen Zerrüttung von auspolymerisierten Kunststoffen, dadurch gekennzeichnet, daß vor dem Aushärten des Polymeren akustische Störkörper mit einem von dem des Polymeren unterschiedlichen Schallwellenwiderstand eingelagert werden, und daß durch selektive Anregung der Störkörper durch akustische Wellen die Polymermatrix zerstört wird.

2. Verfahren nach Anspruch 1, dadurch gekennzeichnet, daß der Kunststoff ein Knochenzement auf der Basis von physiologisch verträglichen Polymeren, insbesondere von aus Präpolymeren und Monomeren hergestellten Polyacrylaten und/oder Polymethacrylaten ist.

3. Verfahren nach Anspruch 1 oder 2, dadurch gekennzeichnet, daß der Schallwellenwiderstand der akustischen Störkörper sich von dem des Polymeren zumindest um den Faktor 1,5 unterscheidet.

4. Verfahren nach mindestens einem der Ansprüche 1—3, dadurch gekennzeichnet, daß die akustischen Störkörper keine scharfen Kanten oder Spitzen haben.

5. Verfahren nach mindestens einem der Ansprüche 1—4, dadurch gekennzeichnet, daß die akustischen Störkörper als Rotationsellipsoide, Zylinderscheiben mit abgerundeten Kanten oder Kugeln ausgebildet sind.

6. Verfahren nach mindestens einem der Ansprüche 1—5, dadurch gekennzeichnet, daß die akustischen Störkörper als Fäden, Geflecht, Gewebe, Folie oder Platte ausgebildet sind.

7. Verfahren nach mindestens einem der Ansprüche 1—6, dadurch gekennzeichnet, daß die akustischen Störkörper aus einem biokompatiblen Material bestehen, dessen Schallwellenwiderstand größer ist als der des Polymeren, wie zum Beispiel $TiAl_5Fe_{2,5}$, CoCrMo, Ta oder $Al_2O_3$.

8. Verfahren nach Anspruch 1, dadurch gekennzeichnet, daß die akustischen Störkörper aus einem Material bestehen, dessen Schallwellenwiderstand kleiner als der des Polymeren ist, wie zum Beispiel Gasblasen, die gezielt bei der Polymerisation freigesetzt wurden.

9. Verfahren nach mindestens einem der Ansprüche 1—8, dadurch gekennzeichnet, daß die Störkörper eine Dicke von etwa 0,01 bis etwa 2 mm besitzen.

10. Verfahren nach mindestens einem der Ansprüche 1—9, dadurch gekennzeichnet, daß die Störkörper in einer Menge von etwa 0,5 bis etwa 20 Vol.% enthalten sind.

11. Verfahren nach mindestens einem der Ansprüche 1—10, dadurch gekennzeichnet, daß als Schallwellen extrakorporal erzeugte fokussierte Stoßwellen eingesetzt werden.

12. Verfahren nach mindestens einem der Ansprüche 1—10, dadurch gekennzeichnet, daß als Schallwellen Ultraschall mit auf die Eigenfrequenz der Störkörper abgestimmter Frequenz eingesetzt wird.

13. Knochenzement auf der Basis von physiologisch verträglichen Polymeren, insbesondere von aus Präpolymeren und Monomeren hergestellten Polyacrylaten und/oder Polymethacrylaten, dadurch gekennzeichnet, daß in das Polymere 0,5 bis 20 Vol.% akustischer Störkörper einer Ducke von 0,01—2 mm aus einem körperverträglichen Metall eingelagert sind.

14. Knochenzement nach Anspruch 13, dadurch gekennzeichnet, daß die Störkörper aus TiAl$_5$Fe$_{2,5}$, CoCrMo oder Tantal bestehen.

**Revendications**

1. Procédé de désagrégation mécanique de matières synthétiques polymérisées, caractérisé en ce qu'avant le durcissement du polymère on incorpore des corps parasites acoustiques ayant une résistance aux ondes sonores différente de celle du polymère, et en ce que par excitation sélective des corps parasites par des ondes acoustiques on détruit la matrice de polymère.

2. Procédé selon la revendication 1, caractérisé en ce que la matière synthétique est un ciment osseux à base de polymères physiologiquement acceptables, en particulier de polyacrylates et/ou polyméthacrylates préparés à partir de prépolymères et de monomères.

3. Procédé selon la revendication 1 ou 2, caractérisé en ce que la résistance aux ondes sonores des corps parasites acoustiques se différencie de celle du polymère au moins d'un facteur 1,5.

4. Procédé selon au moins l'une des revendications 1—3, caractérisé en ce que les corps parasites acoustiques sont dépourvus d'arêtes ou pointes aiguës.

5. Procédé selon au moins l'une des revendications 1—4, caractérisé en ce que les corps parasites acoustiques ont une forme d'ellipsoïdes de rotation, de disques de cylindres à bords arrondis ou de sphères.

6. Procèdè selon au moins l'une des revendications 1—5, caractérisé en ce que les corps parasites acoustiques sont formés en fils, treillis, tissus, feuilles ou plaques.

7. Procédé selon au moins l'une des revendications 1—6, caractérisé en ce que les corps parasites acoustiques se composent d'une matière biocompatible dont la résistance aux ondes sonores est supérieure à celle du polymère, comme par exemple TiAl$_5$Fe$_{2,5}$, CoCrMo, Ta ou Al$_2$O$_3$.

8. Procédé selon la revendication 1, caractérisé en ce que les corps parasites acoustiques se composent d'une matière dont la résistance aux ondes sonores est inférieure à celle du polymère, comme par exemple les bulles de gaz, qui sont libérées de façon orientée dans lors de la polymérisation.

9. Procédé selon au moins l'une des revendications 1—8, caractérisé en ce que les corps parasites possèdent une épaisseur d'environ 0,01 à environ 2 mm.

10. Procédé selon au moins l'une des revendications 1—9, caractérisé en ce que les corps parasites sont contenus en une quantité d'environ 0,5 à environ 20% en volume.

11. Procédé selon au moins l'une des revendications 1—10, caractérisé en ce qu'on utilise comme ondes sonores des ondes de choc concentrées produites de façon extracorporelle.

12. Procédé selon au moins l'une des revendication 1—10, caractérisé en ce qu'on utilise comme ondes sonores des ultra-sons à fréquence réglée sur la fréquence propre des corps parasites.

13. Ciment osseux à base de polymères physiologiquement compatibles, en particulier de polyacrylates et/ou polyméthacrylates préparés à partir de prépolymères et de monomères, caractérisé en ce que dans le polymère sont incorporés de 0,5 à 20% en volume de corps parasites acoustiques d'une épaisseur de 0,01—2 mm faits d'un métal corporellement acceptable.

14. Ciment osseux selon la revendication 13, caractérisé en ce que les corps parasites se composent de TiAl$_5$Fe$_{2,5}$, CoCrMo ou tantale.

**Claims**

1. Process for mechanically disrupting fully polymerized plastics, characterized in that acoustic perturbation elements having an acoustic resistance differing from that of the polymer are incorporated, before the polymer is fully cured, and the polymer matrix is destroyed by selective excitation of the perturbation elements by means of acoustic waves.

2. Process according to Claim 1, characterized in that the plastic is a bone cement based on physiologically tolerated polymers, in particular based on polyacrylates and/or polymethacrylates prepared from prepolymers and monomers.

3. Process according to Claim 1 or 2, characterized in that the acoustic resistance of the acoustic perturbation elements differs from that of the polymer by a factor of at least 1.5.

4. Process according to at least one of Claims 1—3, characterized in that the acoustic perturbation elements do not have any sharp edges or points.

5. Process according to at least one of Claims 1—4, characterized in that the acoustic perturbation elements are formed as ellipsoids of revolution, as cylindrical discs with rounded edges or as spheres.

6. Process according to at least one of Claims 1—5, characterized in that the acoustic perturbation elements are formed as threads, a braiding, a fabric, a foil or a plate.

7. Process according to at least one of Claims 1—6, characterized in that the acoustic perturbation elements consist of a biocompatible material, the acoustic resistance of which is greater than that of the polymer, such as, for example, $TiAl_5Fe_{2.5}$, CoCrMo, Ta or $Al_2O_3$.

8. Process according to Claim 1, characterized in that the acoustic perturbation elements consist of a material, the acoustic resistance of which is smaller than that of the polymer, such as, for example, gas bubbles released in a controlled manner during the polymerization.

9. Process according to at least one of Claims 1—8, characterized in that the perturbation elements have a thickness from about 0.01 to about 2 mm.

10. Process according to at least one of Claims 1—9, characterized in that the perturbation elements are present in a quantity from about 0.5 to about 20% by volume.

11. Process according to at least one of Claims 1—10, characterized in that extracorporeally generated, focused shock waves are employed as the sound waves.

12. Process according to at least one of Claims 1—10, characterized in that ultrasonic waves having a frequency tuned to the characteristic frequency of the perturbation elements are employed as the sound waves.

13. Bone cement based on physiologically tolerated polymers, in particular based on polyacrylates and/or polymethacrylates prepared from prepolymers and monomers, characterized in that 0,5 to 20% by volume of acoustic perturbation elements of a thickness of 0.01—2 mm and of a metal tolerated by the body are incorporated.

14. Bone cement according to Claim 13, characterized in that the perturbation elements consist of $TiAl_5Fe_{2.5}$, CoCrMo or tantalum.